# EUROPEAN PATENT APPLICATION

(11) **EP 4 119 045 A1**
(43) Date of publication of application: **18.01.2023**
(21) Application number: 22150855.9
(22) Date of filing: 11.01.2022
(51) Int. Cl.: A61B 5/0537, A61B 5/00, A61N 1/32

(54) **SKIN CARE DEVICE THAT CAN CHECK IMPROVED SKIN BASED ON BIOMETRIC INFORMATION**

(30) Priority: 16.07.2021 KR 20210093198
(71) Applicant: Saerom Biotech Co., Ltd., Incheon 22376 (KR)
(72) Inventor: PARK, Saerom, 10056 Gimpo-si, Gyeonggi-do (KR)
(74) Representative: M. Zardi & Co S.A.

(57) **Abstract**

The present invention relates to a skin care device that maximizes effects of cosmetics by generating a microcurrent on the skin and allows a user to directly recognize effects of the skin care device by checking skin condition using a configuration capable of detecting biometric information.

The skin care device comprises: a main body unit equipped with a power button; a current rod installed on the main body unit and transmitting a microcurrent to a user's skin; a control unit installed inside the main body unit, supplying and controlling the microcurrent to the current rod based on an operation signal of the power button, and a sensor unit installed on the main body unit and measuring the user's skin information, wherein the skin care device informs skin condition of the user in real time based on the skin information measured by the sensor unit.

## Description

### FIELD OF THE INVENTION

The present invention relates to a skin care device that can check improved skin based on biometric information, and more particularly, to a skin care device that maximizes effects of cosmetics applied to the skin by generating a microcurrent on the skin and allows a user to directly recognize effects of the skin care device by checking skin condition using a configuration capable of detecting biometric information.

### BACKGROUND OF THE INVENTION

The primary causes of skin aging can be largely divided into lack of moisture in the skin, active oxygen, and ultraviolet radiation. Lack of moisture causes wrinkles and excessive dead skin cells, reduces elasticity of skin, and causes various skin problems such as acne. In addition, active oxygen accelerates aging by oxidizing collagen. Furthermore, ultraviolet rays destroy elastic fibers of the skin, causing wrinkles, darkening the skin tone such as pigmentation, and causing various skin problems.

Accordingly, various functional cosmetics have been developed to improve the skin for people of all ages with different skin types. Specifically, cosmetics specialized in specific functions, such as skin nourishment, keratin improvement, skin moisturizing, oil-water balance control, elasticity improvement, wrinkle improvement, skin lightening, and UV protection, have been released and used.

However, since a thick protein protective layer is formed between the epidermis and the dermis in the skin, the nutrition of cosmetics cannot penetrate deep into the dermis layer, and there are limitations to skin care using cosmetics.

Meanwhile, many people use specialized service such as a skin care treatment room where various devices are used to examine and improve the skin condition. This skin care treatment room uses a device such as a microscope showing an enlarged image of the skin to identify the skin condition and inform the clients of their skin condition.

However, since the service provider simply checks the skin condition with the naked eye, it is difficult for a client to obtain an objective evaluation on their skin condition, and each skin specialist may have a different opinion on the same skin. In addition, since those skin care treatment rooms increase the profits by providing their service using expensive cosmetics, clients have high cost burden and customers confidence may decline.

In addition, since clients have to visit skin care treatment rooms to see the skin condition, they cannot check the skin condition frequently, and thus, it is difficult to determine what kind of environmental change factor has affected the skin condition.

Meanwhile, in order to improve the skin, a skin care device that can be easily used at home has been developed and used. The device is designed to generate low-frequency, ultrasonic waves, heat, and negative ions, to clean and remove dead skin cells by operating a brush, or to have a skin massage function.

However, with this conventional skin care device, there are limitations that users only obtain their own subjective evaluation on the improvement of the skin conditions, and it is impossible for the users to know the objective efficacy of the device. In addition, since users do not know the degree of the skin improvement after the use of the device, it is impossible to perform customized skin treatment.

### Prior Art

(Patent Document 0001) Korean Patent Registration No. 10-2055608
(Patent Document 0002) Korean Patent Registration No. 10-2190159 Technical Problem

The present invention has been devised to solve the above problems, and an object of the present invention is to provide a skin care device that can check improved skin based on biometric information. Specifically, the object of the skin care device is to maximize effects of cosmetics by generating a microcurrent on the skin and helping liquid cosmetics to penetrate deeply into the skin, and to increase the reliability by including a configuration that can determine the skin condition to objectively evaluate the degree of improvement after the use of the device and allowing a user to directly know the effects of the device.

In addition, another object of the present invention is to provide a skin care device that can suggest a customized skin treatment method necessary for each user by checking the overall skin condition of the user, which can bring the same effect as receiving a skin improvement solution from a skin expert.

### SUMMARY OF THE INVENTION

A skin care device of the present invention comprises: a main body unit equipped with a power button; a current rod installed on the main body unit and transmitting a microcurrent to a user's skin; a control unit installed inside the main body unit, supplying and controlling the microcurrent to the current rod based on an operation signal of the power button, and a sensor unit installed on the main body unit and measuring the user's skin information, wherein the skin care device informs skin condition of the user in real time based on the skin information measured by the sensor unit.

In addition, the sensor unit includes: a current sensor sending a microcurrent to the skin and then measuring a value of current returned from the skin, and an image sensor capturing the user's skin at a close distance, wherein the skin care device further comprises an operation unit classifying and analyzing the measured value obtained from the sensor unit for each of items of moisture content, oil content, skin elasticity, skin aging, and lightness of the skin and calculating each value for each of the items based on the measured value.

Furthermore, the skin care device further comprises a comparison and determination unit that provides information on the item of which the calculated value is compared with and exceeds a pre-stored threshold value.

Moreover, the skin care device further comprises a display unit installed on the main body unit to display information of skin condition of the user.

In addition, the skin care device further comprises: a short-distance communication module installed on the main body unit, and a user terminal performing communication through the short-distance communication module, wherein the skin care device displays information of skin condition of the user through an application installed on the user terminal.

In this case, the user terminal includes: a cosmetic data loading unit loading a plurality of cosmetic information stored in a server, and a cosmetic selection unit selecting at least one or more cosmetics type by matching the skin condition of the user provided to the user terminal to an appropriate cosmetic type, wherein the user terminal provides information of the selected cosmetic type to the user.

### Advantageous Effects

The skin care device of the present invention can maximize effects of cosmetics by generating a microcurrent on the skin and helping liquid cosmetics to penetrate deeply into the skin, and can increase the reliability by including a configuration that can determine the skin condition to objectively evaluate the degree of improvement after the use of the device and allowing a user to directly know the effects of the device.

In addition, the skin care device of the present invention can suggest a customized skin treatment method necessary for each user by checking the overall skin condition of the user, which can bring the same effect as receiving a skin improvement solution from a skin expert.

### BRIEF DESCRIPTION OF THE DRAWINGS

Fig. 1 is a drawing illustrating a body of a skin care device that can check improved skin based on biometric information according to the present invention.
Fig. 2 is a drawing illustrating an overall configuration of the first embodiment of a skin care device according to the present invention.
Fig. 3 is a drawing illustrating a sensor unit and an operation unit of a skin care device according to the present invention.
Fig. 4 is a drawing illustrating an overall configuration of the second embodiment of a skin care device according to the present invention.
Fig. 5 is a drawing illustrating a cosmetic data loading unit and a cosmetic selection unit of a skin care device according to the present invention.

### DETAILED DESCIPTION EMBODIEMENTS OF THE INVENTIONS

Hereinafter, preferred embodiments of the present invention will be described in detail with reference to the accompanying drawings. In the description of the present invention, if a detailed description of related known functions or configurations may unnecessarily obscure the gist of the present invention, the detailed description thereof will be omitted.

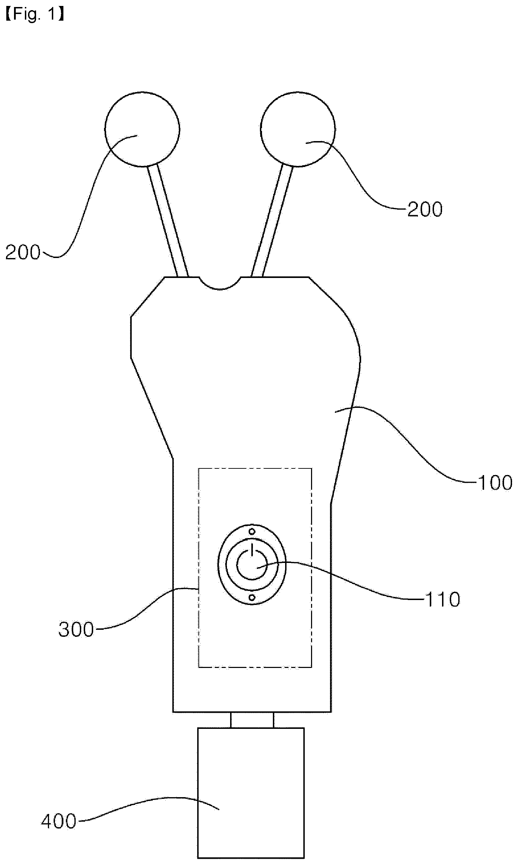

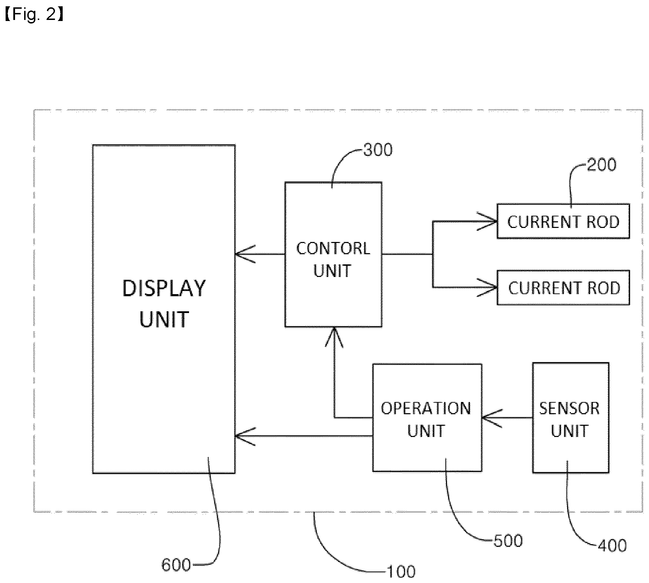

Referring to FIGS. 1 and 2, a skin care device that can check skin improved based on biometric information of the present invention comprises: a main body unit 100 provided with a power button 110; a current rod 200 installed on the main body unit 100 and transmitting a microcurrent to a user's skin; a control unit 300 installed inside the main body unit 100, supplying the microcurrent to the current rod 200 and controlling the microcurrent based on an operation signal of the power button 110, and a sensor unit 400 installed on the main body unit 100 and measuring the user's skin information, wherein the skin care device informs skin condition of the user in real time based on the information measured by the sensor unit 400.

As shown in FIG. 1, the skin care device of the present invention allows a microcurrent to be transmitted to the skin through a pair of current rods 200 installed on the main body unit 100. A current generated from the current rods 200 generates the microcurrent having a certain intensity similar to that of an electric current in the human body. The microcurrent stimulates the internal cellular tissue of the skin to increase the elasticity of the tissue, and ionizes the skin to help skin care cosmetics to penetrate deeply into the skin to increase the absorption rate of the cosmetics and maximize unique effects of the cosmetics. The skin care cosmetics mentioned above may be for skin nourishment, keratin improvement, moisturizing, oil-water balance control, elasticity improvement, wrinkle improvement, skin lightening, UV protection, and the like.

In addition, the skin care device further comprises a configuration generating vibration inside the current rod 200, which further enhances the skin massage effect.

The feature of the present invention is that the user can directly and objectively grasps the degree of improvement after use of the skin care device, thereby increasing the reliability of the device and allowing the users to easily find certain cosmetics suitable for them.

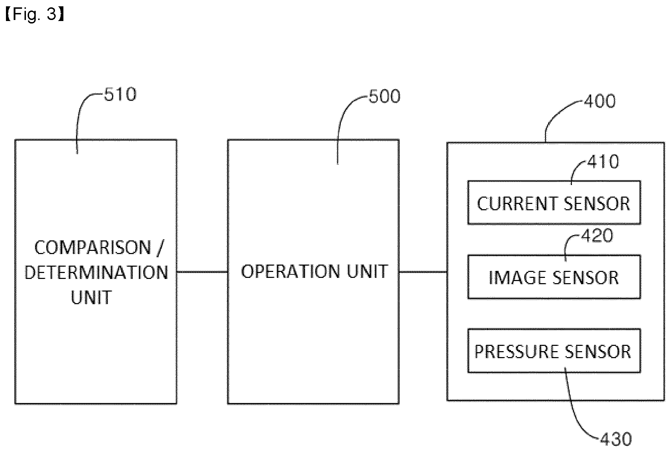

The skin care device of the present invention can obtain biometric information related to the user's skin through the sensor unit 400 installed on the main body unit 100. As shown in FIG. 3, the sensor unit 400 may include a current sensor 410 sending a microcurrent to the skin and then measuring a current returned from the skin, and an image sensor 420 capturing the user's skin at a close distance.

In this case, the skin care device may further comprise an operation unit 500 classifying and analyzing the measured value obtained from the sensor unit 400 for each item of moisture content, oil content, skin elasticity, skin aging, and lightness of the skin and calculating each value for each item based on the measured value.

The current sensor 410 of the sensor unit 400 can measure the moisture content of the skin. As a specific example, since the stratum corneum of the epidermis has high resistance to electricity, the skin care device can obtain moisture content of the skin by measuring conductance, which is the reciprocal of resistance based on the principle that resistance decreases under alternating current.

The current sensor 410 may measure oil content of the skin by using the same principle as well as the moisture content. In addition, the current sensor 410 can determine the elasticity of the skin by inversely estimating the measured value of moisture content, or can determine the degree of aging of the skin by inversely estimating the oil content of the skin.

In addition, the image sensor 420 may determine the level of lightness of the skin by analyzing the RGB values of the captured image of the skin. In this case, the image sensor 420 can determine the tone of the skin or whether any skin troubles have occurred.

The skin care device may further comprise a pressure sensor 430 measuring the repulsive force of the skin after applying a certain pressure to the skin. The pressure sensor 430 enables to determine the elasticity of the skin more effectively. The degree of aging or the elasticity of the skin described above can be determined by comprehensively calculating the values obtained from the current sensor 410, the image sensor 420, and the pressure sensor 430.

In this way, the operation unit 500 classifies and analyzes the measured value obtained from the sensor unit 400 for each item of moisture content, oil content, skin elasticity, skin aging, and lightness of the skin and calculates each value for each item based on the measured value. The information for the calculated values is displayed to the user.

As shown in FIG. 2, the skin care device may be configured to include a display unit 600 installed on the main body unit 100 to display the information of the skin condition of the user based on the calculated values. In this case, if the information of the skin condition is provided through the display unit 600 installed on the body unit 100, the operation unit 500 is provided inside the main body unit 100.

In addition, the skin care device of the present invention may further comprise a comparison and determination unit 510 that either displays the calculated values or informs information on any one of the items of which the calculated value is compared with and exceeds a pre-stored threshold value for each item. With the comparison and determination unit 510, the user can easily identify which part needs improvement among the items of moisture content, oil content, skin elasticity, skin aging, and lightness of the skin.

The threshold value may be directly input by a manufacturer, may be applied with an average value of other users, and may be applied with the user's own past calculated value.

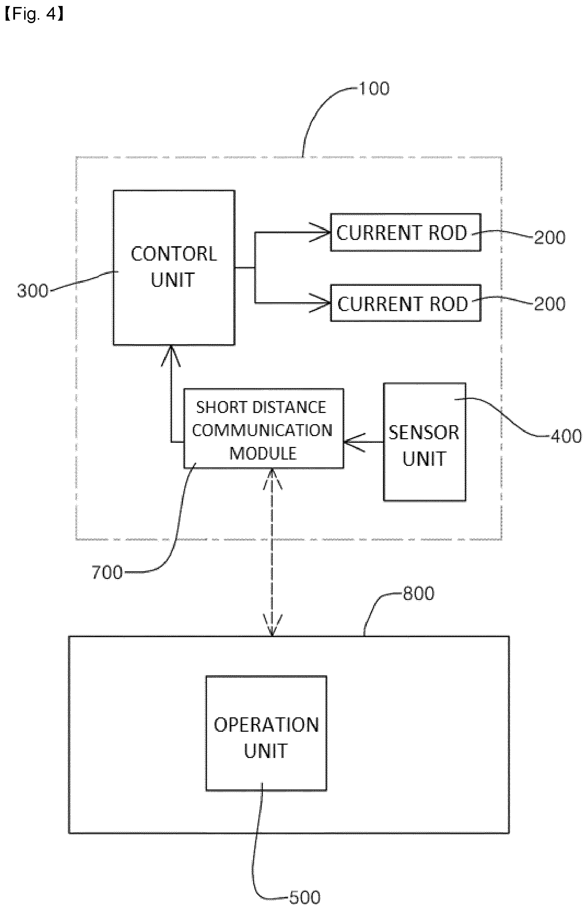

FIG. 4 is another embodiment of the present invention. A skin care device of this embodiment further comprises a short-distance communication module 700 installed on the main body unit 100 and a user terminal 800 performing communication through the short-distance communication module 700. The user terminal 800 may be a device such as a smart phone, and can display information of the skin condition of the user through an application installed on the user terminal 800.

In the above embodiment, the operation unit 500 calculating the moisture content, oil content, skin elasticity, skin aging, and lightness of the skin is not configured in the main body unit 100, but may be configured in the application of the user terminal 800. In this case, the computational efficiency of the operation unit 500 may be further improved.

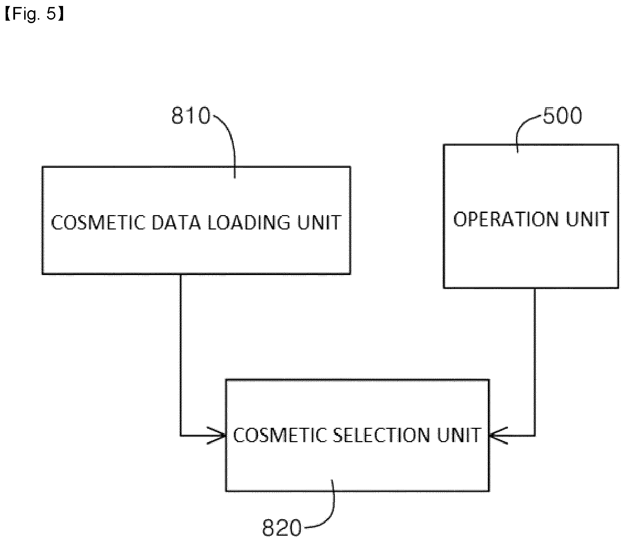

Meanwhile, as shown in FIG. 5, the user terminal 800 in the above embodiment may include a cosmetic data loading unit 810 loading a plurality of cosmetic information stored in a server, and a cosmetic selection unit 820 selecting at least one or more specific cosmetics by matching the skin condition of the user provided to the user terminal 800 to an appropriate cosmetic type, and provide information of the selected cosmetic type to the user.

In other words, a plurality of cosmetic information is stored in the server, and various cosmetic information is loaded through the cosmetic data loading unit 810. Based on the loaded cosmetic information in the cosmetic data loading unit 810, the cosmetic selection unit 820 finds a specific type of cosmetic suitable for the skin condition of the user, and recommends the most appropriate cosmetic to the user.

In addition, the control unit 300 of the present invention is connected to the operation unit 500 and precisely adjusts intensity of the current supplied to the current rod 200 according to the skin condition of the user, so that the current suitable for the skin condition of the user can be transmitted and used. In this case, the control unit 300 can also adjust intensity of vibration of the current rod 200 together.

Although the present invention has been described above with reference to the above embodiments, various modifications are possible within the scope of the technical spirit of the present invention.

## Claims

1. A skin care device that can check skin improved based on biometric information, the skin care device comprises:
a main body unit 100 equipped with a power button 110; a current rod 200 installed on the main body unit 100 and transmitting a microcurrent to a user's skin;
a control unit 300 installed inside the main body unit 100, supplying and controlling the microcurrent to the current rod 200 based on an operation signal of the power button 110, and
a sensor unit 400 installed on the main body unit 100 and measuring the user's skin information,
wherein the skin care device provides information of skin condition of the user in real time based on the skin information measured by the sensor unit 400.

2. The skin care device of claim 1, wherein the sensor unit 400 includes:
a current sensor 410 sending the microcurrent to the skin and then measuring a value of a current returned from the skin, and
an image sensor 420 capturing the user's skin at a close distance,
wherein the skin care device further comprises an operation unit 500 classifying and analyzing a measured value obtained from the sensor unit 400 for each of items of moisture content, oil content, skin elasticity, skin aging, and lightness of the skin and calculating each value for each of the items based on the measured value.

3. The skin care device of claim 2, further comprises a comparison and determination unit 510 that provides information on the item of which the calculated value is compared with and exceeds a pre-stored threshold value.

4. The skin care device of claim 1, further comprises a display unit 600 installed on the main body unit 100 to display information of skin condition of the user.

5. The skin care device of claim 1, further comprises:
a short-distance communication module 700 installed on the main body unit 100, and
a user terminal 800 performing communication through the short-distance communication module 700,
wherein the skin care device displays information of skin condition of the user through an application installed on the user terminal 800.

6. The skin care device of claim 5, wherein the user terminal 800 includes:
a cosmetic data loading unit 810 loading a plurality of cosmetic information stored in a server, and
a cosmetic selection unit 820 selecting at least one or more cosmetics type by matching the skin condition of the user provided to the user terminal 800 to the appropriate cosmetic type,
wherein the user terminal 800 provides information of the selected cosmetic type to the user.
